(19) Europäisches Patentamt
European Patent Office
Office européen des brevets

(11) Numéro de publication : **0 028 279**
**B1**

(12) # FASCICULE DE BREVET EUROPÉEN

(45) Date de publication du fascicule du brevet : **16.02.83**

(21) Numéro de dépôt : **79400780.7**

(22) Date de dépôt : **22.10.79**

(51) Int. Cl.³ : **C 07 D405/04**// A61K31/47, C07D217/02, C07D217/10

(54) **Dérivés de phtalidyl-isoquinoléines, leur préparation et leur application comme médicaments.**

(43) Date de publication de la demande :
**13.05.81 Bulletin 81/19**

(45) Mention de la délivrance du brevet :
**16.02.83 Bulletin 83/07**

(84) Etats contractants désignés :
**AT BE CH DE GB LU NL SE**

(56) Documents cités :
**FR A 1 295 309**
**FR A 2 424 271**
**JOURNAL OF ORGANIC CHEMISTRY, Vol. 37 (1972) Columbus Ohio US « Conversion of (−)-beta-Hydrastine into (−)-Bicuculline and related Phtalideisoquinolines » pages 1879-81**

(73) Titulaire : **CALAIRE CHIMIE S.A.**
**1 quai d'Amérique**
**F-62100 Calais, Pas-de-Calais (FR)**
**LABOREC LABORATOIRE DE RECHERCHES BIOLOGIQUES**
**31 rue de l'Alma**
**F-92600 Asnières, Hauts-de-Seine (FR)**

(72) Inventeur : **Chazerain, Jacques André**
**1 allée du Grand Tulipier**
**Ville d'Avray, Hauts-de-Seine (FR)**
Inventeur : **Cotereau, Hubert Yves**
**10 rue du Longpont**
**Neuilly-sur-Seine, Hauts-de-Seine (FR)**
Inventeur : **Lallouette, Pierre Henri**
**2 rue de Neptune**
**Sannois, Val d'Oise (FR)**
Inventeur : **Legger, Hugues André**
**12 rue de l'Abreuvoir**
**Courbevoie, Hauts-de-Seine (FR)**
Inventeur : **Lepape, Pierre Adolphe Charles**
**54 Avenue Théophile Gautier**
**Paris (FR)**

(74) Mandataire : **Gallochat, Alain**
**SOCIETE D'ETUDES SCIENTIFIQUES ET INDUSTRIELLES DE L'ILE DE FRANCE 46, Boulevard de Latour Maubourg**
**F-75340 Paris Cedex 07 (FR)**

**EP 0 028 279 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Dérivés de phtalidyl-isoquinoléines, leur préparation et leur application comme médicaments.

La présente invention concerne des dérivés de phtalidyl-isoquinoléines, leur préparation et leur application comme médicaments, notamment dans le traitement des manifestations allergiques.

On connaît depuis longtemps la noscapine, alcaloïde utilisé à cause de ses propriétés antitussives. Un dérivé structurellement proche de cette dernière, la tritoqualine (voir le brevet français N° 1 295 309) a été remarquée pour ses propriétés anti-allergiques (Hahn et coll. Arz. Forsch. *20,* 1 490 (1970)).

Il a maintenant été trouvé que de nouveaux composés de ce type ont une activité similaire plus intéressante et peuvent donc trouver une application en thérapeutique.

Les composés de l'invention répondent à la formule :

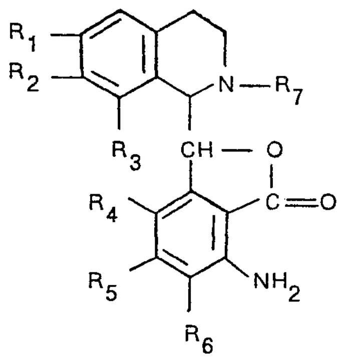

(I)

dans laquelle

$R_1$, $R_2$, $R_4$, $R_5$ et $R_6$ représentent chacun un groupe alcoxy inférieur,

$R_3$ représente un atome d'hydrogène ou un groupe alcoxy inférieur, et

$R_7$ représente un groupe alkyle inférieur, ainsi que leurs diastéréoisomères et leurs sels d'addition à des acides pharmaceutiquement acceptables.

On entend par groupes alkyle et alcoxy inférieurs des groupes ayant au plus 4 atomes de carbone, en particulier les radicaux méthyle et méthoxy ou éthoxy respectivement.

Les composés de formule (I) peuvent être préparés par réduction catalytique ou chimique du dérivé nitré correspondant, de formule

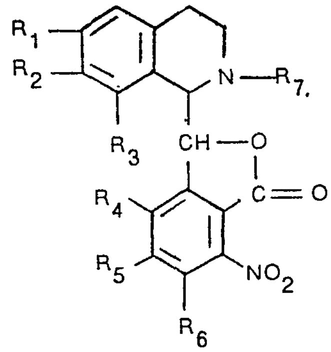

(II)

dans laquelle les divers symboles ont la même signification que dans la formule (I).

Les dérivés nitrés (II) peuvent être préparés par condensation d'un sel d'ammonium d'une dihydro-isoquinoléine substituée (III) et d'un trialcoxy-phtalide (IV) suivant le schéma réactionnel :

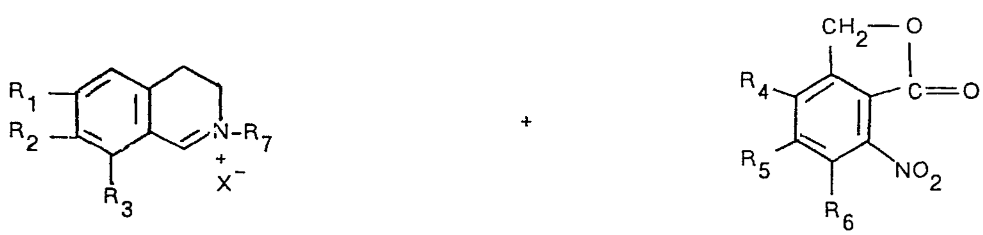

(III), $R_7X$ (IV)

Dans ces formules les divers symboles $R_1$ à $R_7$ ont la même signification que dans la formule (I) et $X^-$ est un anion tel que $OH^-$, $Cl^-$, $Br^-$, $I^-$ ou $CH_3SO_4^-$. La réaction est effectuée en milieu basique et de

préférence dans un alcool inférieur en dessous de son point d'ébullition.

Les dérivés de formule (IV) sont connus d'après le brevet français N° 1 295 309.

Les sels d'ammonium des dihydroxy-isoquinoléines substituées (III, $R_7X$) sont en partie connus et peuvent être préparés de manière classique par addition d'un composé $R_7X$ sur la dihydroxy-isoquinoléine substituée (III). Celle-ci peut être préparée par formylation d'une phényléthylamine substituée suivie d'une cyclisation selon le schéma

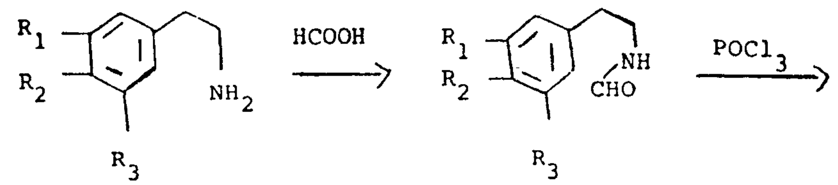


(III)

Dans ces formules les symboles $R_1$, $R_2$ et $R_3$ ont la même signification que ci-dessus. La réaction de formylation est effectuée de manière habituelle à l'aide d'acide formique et d'anhydride acétique à une température allant de 25 à 80 °C. La cyclisation peut être effectuée avec de l'oxychlorure de phosphore à une température allant de 50 à 100 °C.

Les exemples suivants illustrent l'invention.

## Exemple A

Iodure de méthyl-2 triméthoxy-6,7,8 dihydro-3,4 isoquinoléinium (produit intermédiaire III, $R_7X$ ; $R_1$, $R_2$, $R_3 = OCH_3$, $R_7 = CH_3$ et $X = I$)

a) Formyl-2 triméthoxy-3,4,5 phényléthylamine.

A un mélange de 2,185 kg d'acide formique à 98 % et 1,728 kg d'anhydride acétique on ajoute lentement 1,422 kg de triméthoxy-3,45 phényléthylamine, tout en maintenant la température à environ 50°. On maintient le bain réactionnel à cette température pendant environ 3 heures puis on distille le mélange d'acide formique et acétique et l'excès d'anhydride. On utilise le dérivé formylé ainsi obtenu (1, 734 kg) à l'état brut.

b) Triméthoxy-6,7,8 dihydro-3,4 isoquinoléine.

On introduit dans un réacteur le dérivé formylé obtenu ci-dessus et 3 litres de toluène à une température d'environ 70°, on introduit 1,385 kg d'oxychlorure de phosphore et on maintient le mélange réactionnel à cette température pendant environ 2 heures. Ensuite on refroidit au-dessous de 50° et on coule le mélange réactionnel dans 5,1 litres d'eau et 1,8 kg de glace pilée. On sépare la phase aqueuse acide, on alcalinise avec une solution d'hydroxyde de sodium à 40 %, puis on extrait du toluène. On chasse le toluène sous vide et on utilise la triméthoxy-6,7,8 dihydro-3,4 isoquinoléine à l'état brut.

c) Iodure de méthyl-2 triméthoxy-6,7,8 dihydro-3,4 isoquinoléinium.

On introduit dans un réacteur 1,9 kg de triméthoxy-6,7,8 dihydro-3,4 isoquinoléine et 7 litres d'acétone, puis 2,1 kg d'iodure de méthyle en maintenant la température au-dessous de 50°. On chauffe ensuite au reflux pendant 30 minutes, on ajoute à nouveau 200 g d'iodure de méthyle et on poursuit le reflux pendant environ 1 heure.

On refroidit au-dessous de 20°, essore le iodo-méthylate et lave avec un peu d'acétone. On obtient 2,300 kg.

## Exemple 1

Méthyl-2 triméthoxy-6,7,8 [triéthoxy-4,5,6 amino-7 phtalidyl-3]-1 tétrahydro-1,2,3,4 isoquinoléine. (N° de code 458 L).

a) Méthyl-2 triméthoxy-6,7,8 [triéthoxy-4,5,6 nitro-7 phtalidyl-3]-1 tétrahydro-1,2,3,4 isoquinoléine.

Dans 1,240 l de méthanol on introduit 235 g (0,647 mole) d'iodure de méthyl-2 triméthoxy-6,7,8 dihydro-3,4 isoquinoléinium (décrit dans J. Chem. Soc. 1951, 1 150) 201,4 g de triéthoxy-4,5,6 nitro-7

phtalide et 107 g de carbonate de potassium. Après agitation pendant 24 heures à une température inférieure à la température d'ébullition du solvant, on ajoute 730 ml d'eau et on laisse cristalliser. On recueille les cristaux par filtration, on lave à l'eau et après séchage on obtient 270 g de $C_{27}H_{34}O_{10}N_2$. P.F. 116-140°.

b) Méthyl-2 triméthoxy-6,7,8 [triéthoxy-4,5,6 amino-7 phtalidyl-3]-1 tétrahydro-1,2,3,4 isoquinoléine.

On ajoute 270 g du dérivé nitré précédent (a) à 1,570 l d'éthanol et 21 g de nickel de Raney dans un autoclave. On introduit de l'hydrogène sous une pression de 10 bars et on chauffe à température de 65° en maintenant une agitation. Après 1 heure 45, le volume théorique d'hydrogène est absorbé. On refroidit et on filtre la solution. On distille l'éthanol sous vide, on dissout le résidu dans 0,6 l de méthanol, on ajoute 60 g de potasse en pastilles et on chauffe à reflux pendant 1 heure en agitant. On refroidit et laisse cristalliser. On isole 194,5 g. (rendement = 76 %) de méthyl-2 triméthoxy-6,7,8 (triéthoxy-4,5,6 amino-7 phtalidyl-3)-1 tétrahydro-1,2,3,4 isoquinoléine.

Cristaux blancs, de point de fusion : 144° ne présentant qu'une seule tache en chromatographie sur couche mince.

Exemple 2

Méthyl-2 diméthoxy-6,7 [triéthoxy-4,5,6 amino-7 phtalidyl-3]-1 tétrahydro-1,2,3,4 isoquinoléine.

a) Méthyl-2 diméthoxy-6,7 [triéthoxy-4,5,6 nitro-7 phtalidyl-3]-1 tétrahydro-1,2,3,4 isoquinoléine.

Dans 1,5 l de méthanol on introduit en agitant 200 g (0,6 mole) d'iodure de méthyl-2 diméthoxy-6,7 dihydro-3,4 isoquinoléinium (préparé de la même manière que le composé de l'exemple A), 186 g de triéthoxy-4,5,6 nitro-7 phtalide et 100 g de carbonate de potassium. On laisse agiter pendant 24 heures à une température inférieure à la température d'ébullition du solvant, puis on laisse refroidir ; on ajoute 900 ml d'eau et on laisse cristalliser. On filtre, rince avec du méthanol puis de l'eau.

On obtient après séchage : 247 g de cristaux crème de $C_{26}H_{32}O_9N_2$.

b) Méthyl-2 diméthoxy-6,7 [triéthoxy-4,5,6 amino-7 phtalidyl-3]-1 tétrahydro-1,2,3,4 isoquinoléine.

Dans un premier temps on prépare un couple Zn-Cu :

On lave 350 g de poudre de zinc par agitation dans 500 ml de solution d'hydroxyde de sodium à 1 % ; on sépare la solution par décantation puis on lave à deux reprises à l'eau par décantation. A la poudre lavée on ajoute 700 ml de solution préparée en dissolvant 17 g de sulfate cuivrique et 7 ml d'acide sulfurique concentré dans 700 ml d'eau. On laisse agiter pendant 15 minutes, on laisse décanter puis on élimine la solution surnageante.

Dans un deuxième temps on ajoute au couple ainsi préparé 500 ml d'eau, puis on introduit peu à peu tout en agitant, la solution de 167 g du composé nitré obtenu au stade précédent (a) dissout dans 1 litre d'acide acétique. On continue l'agitation pendant 48 heures à la température de 20-25°. On sépare le couple par filtration, on concentre partiellement le filtrat sous vide et on ajoute 2,5 litres d'eau ; on extrait 3 fois au chloroforme.

On réunit les extraits chloroformiques, lave avec une solution de bicarbonate de sodium, puis avec de l'eau. On sèche par agitation avec du sulfate de soude anhydre, puis on concentre sous vide.

On dissout le résidu dans 500 ml de méthanol, on ajoute 50 g de potasse en pastilles et on chauffe à reflux 1 heure en agitant. On refroidit et laisse cristalliser. On isole 115 g de méthyl-2 diméthoxy-6,7 [triéthoxy-4,5,6 amino-7 phtalidyl-3]-1 tétrahydro-1,2,3,4 isoquinoléine. Cristaux blancs. Point de fusion : 143 °C.

Les composés de l'invention ont été soumis à des essais pharmacologiques.

Il a été démontré sur l'animal que les composés de l'invention sont bien supportés aux doses envisagées pour leur utilisation en thérapeutique.

La toxicité a été déterminée de manière habituelle. La DL 50 du composé 458 L chez la souris par voie orale est voisine de 6 g/kg et les essais de toxicité chronique chez la souris ont montré la bonne tolérance de ce produit.

Il a été établi que le composé 458 L possède des propriétés intéressantes, en particulier celle d'inhiber *in vivo* l'action de l'enzyme histidine-décarboxylase freinant ainsi la formation endogène de l'histamine dont le rôle pathologique chez l'animal et chez l'homme est bien connu.

L'activité du composé 458 L a été comparée à celle de la tritoqualine qui est à la connaissance des Demandeurs, le seul produit utilisé en thérapeutique pour cette propriété. La technique de mesure de l'activité de l'histidine décarboxylase de rein de cobaye a été décrite par B. Kosmicki *et al.* (J. Pharmacol. Paris 1974 - *5* - 3 - 331 - 342). Les cobayes ont été sacrifiés cinq heures après l'administration des substances étudiées. Il a été déterminé par rapport aux témoins le pourcentage de réduction de l'activité de l'enzyme du rein des animaux traités. Les résultats obtenus sont rapportés dans le tableau ci-dessous :

| Dose de produit exprimée en mg/kg | Activité exprimée en % d'inhibition de l'enzyme | |
|---|---|---|
| | 458 L | Tritoqualine |
| 10 | 14 | 10 |
| 20 | 21 | 20 |
| 40 | 31 | 20 |
| 60 | 41 | 22 |

On constate donc que l'activité inhibitrice peut s'élever au double de celle du produit de référence.

En fonction de leurs propriétés, les composés de l'invention et en particulier le 458 L peuvent être utilisés dans la prévention et le traitement des manifestations allergiques chez l'animal et chez l'homme (pollinoses, urticaire, eczéma, migraine, prurit...).

Les composés de l'invention sont administrables par voie orale ou rectale à la dose quotidienne de 20 à 500 mg. Ils peuvent se présenter par exemple sous forme de comprimés, dragées ou suppositoires.

Ils peuvent également être administrés en inhalation sous forme d'aérosols de poudre micronisée et éventuellement dilués dans un excipient inerte (mannitol par exemple).

Exemples de formulations

Comprimés

| | |
|---|---|
| Corps 458 L | 50 mg |
| Amidon | 50 mg |
| Lactose | 100 mg |
| Talc | 10 mg |
| Stéarate de magnésium | q.s. |

Aérosol ou nébulisation

| | |
|---|---|
| Corps 458 L | 50 mg |
| Mannitol | 150 mg |

pour une capsule déchirable pour aérosols.

**Revendications** (pour les Etats contractants : BE, CH, DE, GB, LU, NL, SE)

1. Composés répondant à la formule

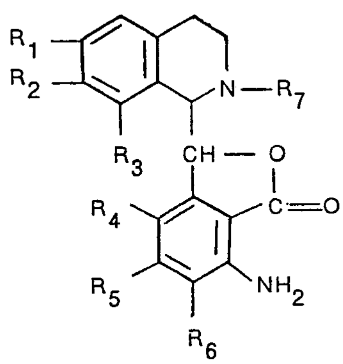

(I)

dans laquelle

$R_1$, $R_2$, $R_4$, $R_5$ et $R_6$ représentent chacun un groupe alcoxy en $C_1$-$C_4$,

$R_3$ représente un atome d'hydrogène ou un groupe alcoxy en $C_1$-$C_4$, et

$R_7$ représente un groupe alkyle en $C_1$-$C_4$, ainsi que leurs diastéréoisomères et leurs sels d'addition à des acides pharmaceutiquement acceptables.

2. Composés selon la revendication 1, répondant à la formule (I) dans laquelle les divers groupes alcoxy représentés par les symboles $R_1$, $R_2$, $R_3$, $R_4$, $R_5$ et $R_6$ sont méthoxy ou éthoxy.

3. Composés selon la revendication 1 ou la revendication 2, répondant à la formule (I) dans laquelle $R_7$ représente un groupe méthyle.

4. La méthyl-2 triméthoxy-6,7,8 [triéthoxy-4,5,6 amino-7 phtalidyl-3]-1 tétrahydro-1,2,3,4 isoquinoléine.

5. La méthyl-2 diméthoxy-6,7 [triéthoxy-4,5,6 amino-7 phtalidyl-3]-1 tétrahydro-1,2,3,4 isoquinoléine.

6. Procédé de préparation des composés de la revendication 1, procédé caractérisé en ce que l'on effectue par condensation d'un sel d'ammonium d'une dihydro-isoquinoléine substituée (III) et d'un trialcoxy-phtalide (IV) suivant le schéma réactionnel :

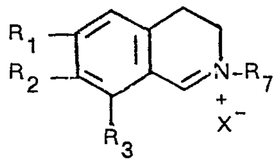

+

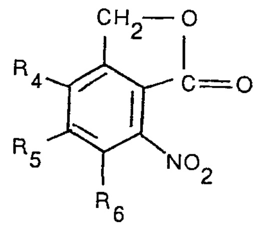

(III), $R_7X$

(IV)

dans lesquelles les divers symboles $R_1$ à $R_7$ ont la même signification que dans la formule (I) et X est un anion tel que $OH^-$, $Cl^-$, $Br^-$, $I^-$ ou $CH_3SO_4^-$ et ensuite on effectue la réduction catalytique ou chimique du dérivé nitré ainsi obtenu de formule :

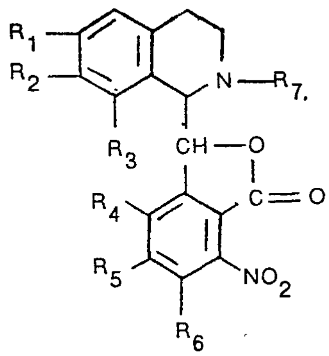

(II)

dans laquelle les divers symboles ont la même signification que dans la formule (I).

7. Composition pharmaceutique contenant un composé spécifié dans l'une quelconque des revendications 1 à 5.

8. Composition pharmaceutique contenant comme substance active le composé spécifié dans la revendication 4.

9. Médicament contenant le composé spécifié dans la revendication 4.

**Revendications** (pour l'Etat contractant AT)

1. Procédé de préparation des composés répondant à la formule :

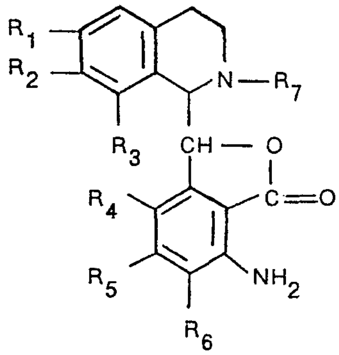

(I)

dans laquelle

$R_1$, $R_2$, $R_4$, $R_5$ et $R_6$ représentent chacun un groupe alcoxy inférieur ($C_1$-$C_4$),

$R_3$ représente un atome d'hydrogène ou un groupe alcoxy inférieur ($C_1$-$C_4$), et

$R_7$ représente un groupe alkyle inférieur ($C_1$-$C_4$) ainsi que leurs diastéréoisomères et leurs sels d'addition à des acides pharmaceutiquement acceptables, procédé caractérisé en ce que l'on effectue

par condensation d'un sel d'ammonium d'une dihydro-isoquinoléine substituée (III) et d'un trialcoxy-phtalide (IV) suivant le schéma réactionnel :

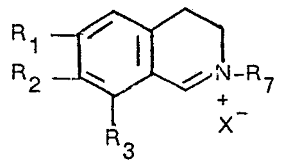

+

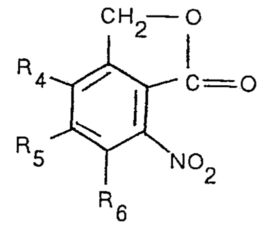

(III), $R_7X$

(IV)

dans lesquelles les divers symboles $R_1$ à $R_7$ ont la même signification que dans la formule (I) et $X^-$ est un anion tel que $OH^-$, $Cl^-$, $Br^-$, $I^-$ ou $CH_3SO_4^-$ et ensuite on effectue la réduction catalytique ou chimique du dérivé nitré ainsi obtenu de formule :

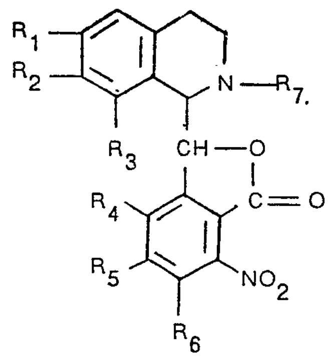

(II)

dans laquelle les divers symboles ont la même signification que dans la formule (I).

2. Procédé de préparation selon la revendication 1, dans lequel les divers groupes alcoxy représentés par les symboles $R_1$, $R_2$, $R_3$, $R_4$, $R_5$, $R_6$ sont méthoxy ou éthoxy.

3. Procédé de préparation selon les revendications 1 ou 2 dans lequel $R_7$ est un groupe méthyle.

4. Procédé de préparation selon les revendications 2 et 3 dans lequel $R_1$, $R_2$ et $R_3$ sont méthoxy, $R_4$, $R_5$, $R_6$ sont éthoxy et $R_7$ est méthyle.

5. Procédé de préparation selon les revendications 2 et 3 dans lequel $R_1$ et $R_2$ sont méthoxy, $R_3$ est hydrogène, $R_4$, $R_5$ et $R_6$ sont éthoxy et $R_7$ est méthyle.

**Claims** (for the Contracting States : BE, CH, DE, GB, LU, NL, SE)

1. Compounds corresponding to the formula

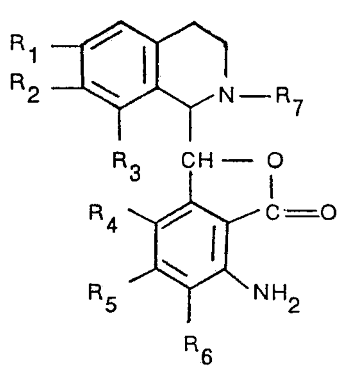

(I)

wherein

$R_1$, $R_2$, $R_4$, $R_5$ and $R_6$ each represent a $C_1$-$C_4$ alkoxy group,

$R_3$ represents a hydrogen atom or a $C_1$-$C_4$ alkoxy group, and

$R_7$ represents a $C_1$-$C_4$ alkyl group, and their diastereoisomers and their pharmaceutically acceptable acid addition salts.

2. Compounds according to claim 1 corresponding to the formula (I), wherein the various alkoxy groups represented by the symbols $R_1$, $R_2$, $R_3$, $R_4$, $R_5$ and $R_6$ are methoxy or ethoxy.

3. Compounds according to claim 1 or claim 2 corresponding to the formula (I), wherein $R_7$ represents a methyl group.

4. 2-Methyl 6,7,8-trimethoxy 1-[4,5,6-triethoxy 7-amino 3-phthalidyl]1,2,3,4-tetrahydro-isoquinoline.

5. 2-Methyl 6,7-dimethoxy 1-[4,5,6-triethoxy 7-amino 3-phthalidyl]1,2,3,4-tetrahydro-isoquinoline.

6. A process for the preparation of the compounds of claim 1, characterised by effecting condensation of an ammonium salt of a substituted dihydro-isoquinoline (III) and a trialkoxy-phthalide (IV) in accordance with the following reaction diagram :

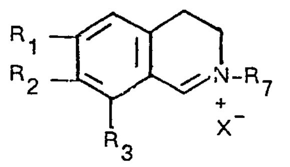

+

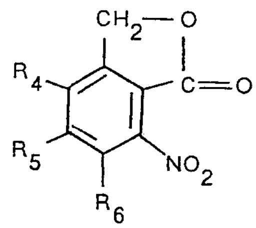

(III), $R_7X$ (IV)

wherein the various symbols $R_1$ to $R_7$ are of the same significance as in the formula (I) and X is an anion such as $OH^-$, $Cl^-$, $Br^-$, $I^-$ or $CH_3SO_4^-$ and then effecting catalytic or chemical reduction of the nitro derivative which is thus produced, having the following formula :

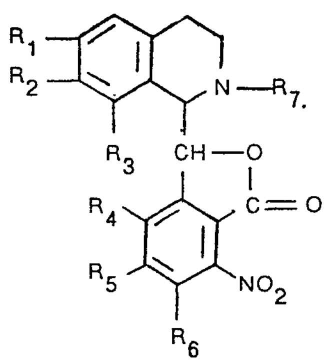

(II)

wherein the various symbols are of the same significance as in formula (I).

7. A pharmaceutical composition containing a compound as specified in any one of claims 1 to 5.

8. A pharmaceutical composition containing as active substance the compound specified in claim 4.

9. A medicament containing the compound specified in claim 4.

**Claims** (for the Contracting State AT)

1. A process for the preparation of compounds corresponding to the formula (I)

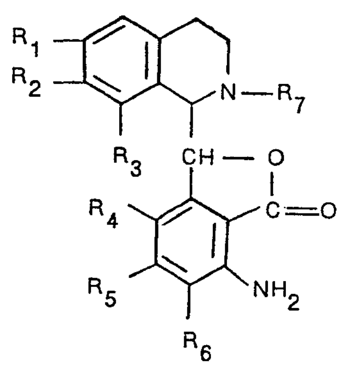

(I)

wherein

$R_1$, $R_2$, $R_4$, $R_5$ and $R_6$ each represent a $C_1$-$C_4$ alkoxy group,

$R_3$ represents a hydrogen atom or a $C_1$-$C_4$ alkoxy group, and

$R_7$ represents a $C_1$-$C_4$ alkyl group, their diastereoisomers, and their pharmaceutically acceptable acid addition salts, characterised by effecting condensation of an ammonium salt of a substituted dihydro-isoquinoline (III) and a trialkoxy-phthalide (IV) in accordance with the following reaction diagram :

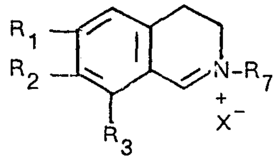

+

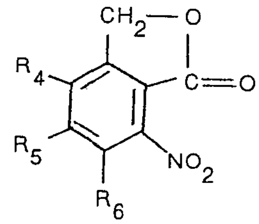

(III), $R_7X$ (IV)

wherein the various symbols $R_1$ to $R_7$ are of the same significance as in the formula (I) and X is an anion such as $OH^-$, $CL^-$, $Br^-$, $I^-$ or $CH_3SO_4^-$ and then effecting catalytic or chemical reduction of the nitro derivative which is thus produced, having the following formula :

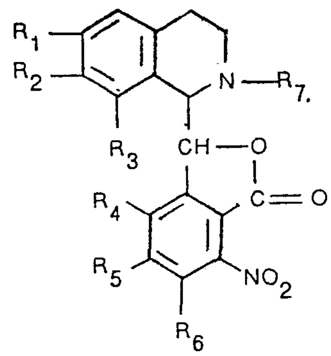

(II)

wherein the various symbols are of the same significance as in formula (I).

2. A process for the preparation of compounds according to claim 1, characterised in that the symbols $R_1$, $R_2$, $R_3$, $R_4$, $R_5$ and $R_6$ represent methoxy or ethoxy groups.

3. A process for the preparation of compounds according to claims 1 or 2, in which $R_7$ represents a methyl group.

4. A process for the preparation of compounds according to claims 2 or 3, in which $R_1$, $R_2$ and $R_3$ represent a methoxy group, $R_4$, $R_5$, and $R_6$ represent an ethoxy group, and $R_7$ represents a methyl group.

5. A process for the preparation of compounds according to claims 2 or 3, characterised in that $R_1$ and $R_2$ represent a methoxy group, $R_3$ is hydrogen, $R_4$, $R_5$ and $R_6$ represent an ethoxy group, and $R_7$ represents a methyl group.

**Ansprüche** (für die Vertragsstaaten : BE, CH, DE, GB, LU, NL, SE)

1. Verbindungen der Formel

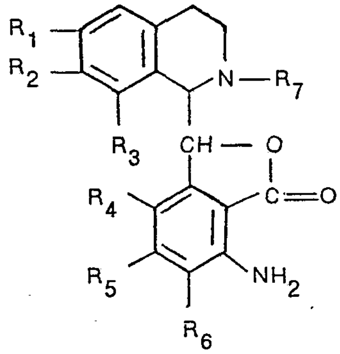

(I)

in der

$R_1$, $R_2$, $R_4$, $R_5$ und $R_6$ jeweils eine $C_1$-$C_4$-Alkoxygruppe,

$R_3$ ein Wasserstoffatom oder eine $C_1$-$C_4$-Alkoxygruppe und

$R_7$ eine $C_1$-$C_4$-Alkylgruppe bedeuten, sowie ihre Diastereoisomeren und Additionssalze mit pharmazeutisch verträglichen Säuren.

2. Verbindungen nach Anspruch 1 der Formel (I), in der die verschiedenen, durch die Symbole $R_1$, $R_2$, $R_3$, $R_4$, $R_5$ und $R_6$ gekennzeichneten Alkoxygruppen Methoxy- oder Ethoxygruppen sind.

3. Verbindungen nach Anspruch 1 oder 2 der Formel (I), in der $R_7$ eine Methylgruppe bedeutet.

4. Methyl-2-trimethoxy-6,7,8-[triethoxy-4,5,6-amino-7-phthalidyl-3]-1-tetrahydro-1,2,3,4-isochinolin.

5. Methyl-2-dimethoxy-6,7-[triethoxy-4,5,6-amino-7-phthalidyl-3]-1-tetrahydro-1,2,3,4-isochinolin.

6. Verfahren zur Herstellung der Verbindungen nach Anspruch 1, dadurch gekennzeichnet, daß man ein Ammoniumsalz eines substituierten Dihydroisochinolins (III) und ein Trialkoxyphthalid (IV) gemäß dem Reaktionsschema

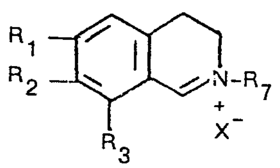

+

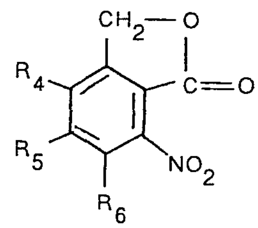

(III), $R_7X$ (IV)

wobei die verschiedenen Symbole $R_1$-$R_7$ die gleiche Bedeutung wie in der Formel (I) aufweisen und $X^-$ ein Anion, z. B. $OH^-$, $Cl^-$, $Br^-$, $J^-$ oder $CH_3SO_4^-$ bedeutet, kondensiert und anschließend das so erhaltene nitrierte Derivat der Formel

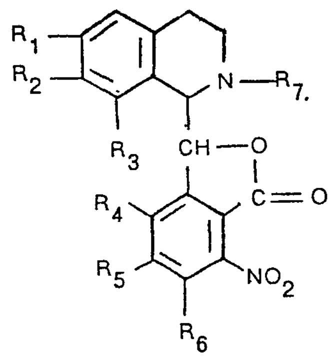

(II)

in der die verschiedenen Symbole die gleiche Bedeutung wie in der Formel (I) aufweisen, katalytisch oder chemisch reduziert.

7. Pharmazeutische Zusammensetzung, enthaltend eine Verbindung nach einem der Ansprüche 1-5.

8. Pharmazeutische Zusammensetzung, enthaltend die Verbindung nach Anspruch 4 als Wirkstoff.

9. Medikament, enthaltend die Verbindung nach Anspruch 4.

**Ansprüche** (für den Vertragsstaat AT)

1. Verfahren zur Herstellung von Verbindungen der Formel

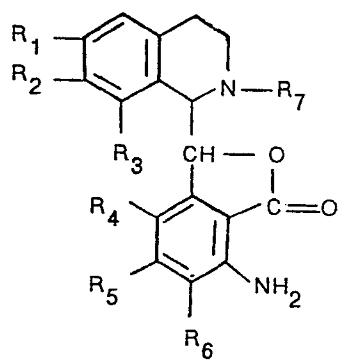

(I)

in der

$R_1$, $R_2$, $R_4$, $R_5$ und $R_6$ jeweils eine niedrige Alkoxygruppe,

$R_3$ ein Wasserstoffatom oder eine niedrige Alkoxygruppe und

$R_7$ eine niedrige Alkylgruppe bedeuten, sowie der Diastereoisomeren und der Additionssalze mit pharmazeutisch verträglichen Säuren derselben, dadurch gekennzeichnet, daß man ein Ammoniumsaltz eines substituierten Dihydroisochinolins (III) und ein Trialkoxyphthalid (IV) gemäß dem Reaktionsschema

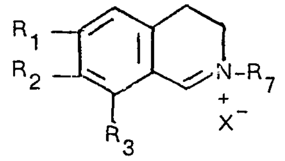

+

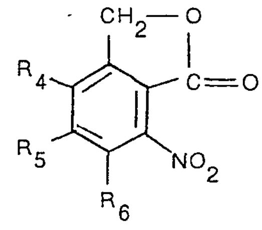

(III), $R_7X$ (IV)

wobei die verschiedenen Symbole $R_1$-$R_7$ die gleiche Bedeutung wie in der Formel (I) aufweisen und $X^-$ ein Anion, z. B. $OH^-$, $Cl^-$, $Br^-$, $J^-$ oder $CH_3SO_4^-$ bedeutet, kondensiert und anschließend das so erhaltene nitrierte Derivat der Formel

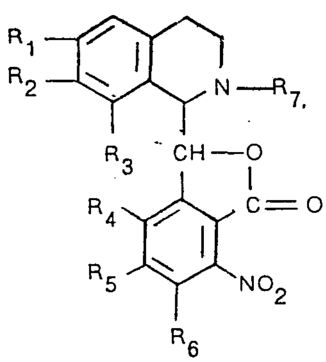

(II)

in der die verschiedenen Symbole die gleiche Bedeutung wie in der Formel (I) aufweisen, katalytisch oder chemisch reduziert.

2. Verfahren zur Herstellung der Verbindung nach Anspruch 1, dadurch gekennzeichnet, daß die unterschiedlichen, durch die Symbole $R_1$, $R_2$, $R_3$, $R_4$, $R_5$ und $R_6$ wiedergegebenen Gruppen Methoxy- oder Ethoxygruppen sind.

3. Verfahren zur Herstellung der Verbindung nach Anspruch 1 oder 2, dadurch gekennzeichnet, daß $R_7$ eine Methylgruppe ist.

4. Verfahren zur Herstellung der Verbindungen nach Anspruch 2 oder 3, dadurch gekennzeichnet, daß $R_1$, $R_2$ und $R_3$ Methoxygruppen, $R_4$, $R_5$ und $R_6$ Ethoxygruppen und $R_7$ eine Methylgruppe bedeuten.

5. Verfahren zur Herstellung der Verbindungen nach Anspruch 2 oder 3, dadurch gekennzeichnet, daß $R_1$ und $R_2$ Methoxygruppen, $R_3$ Wasserstoff, $R_4$, $R_5$ und $R_6$ Ethoxygruppen und $R_7$ eine Methylgruppe bedeuten.